# EUROPÄISCHE PATENTANMELDUNG

(11) **EP 4 834 380 A1**
(43) Veröffentlichungstag der Anmeldung: **04.11.2026**
(21) Anmeldenummer: 26176025.0
(22) Anmeldetag: 30.04.2026
(51) Int. Cl.: A61L 27/36, A61K 8/98, A61M 37/00

(54) **AUTOLOGES NANOFETT IN EINEM SYNERGISTISCHEN MULTILAYER APPLIKATIONSKONZEPT ZUR ÄSTHETISCHEN UND REGENERATIVEN HAUTVERJÜNGUNG, INSBESONDERE ZUR GESICHTSVERJÜNGUNG**

(30) Priorität: 30.04.2025 CH 4722025; 27.06.2025 CH 7102025; 27.08.2025 CH 9712025; 18.11.2025 CH 13292025
(71) Anmelder: Dr. Dragieva Aesthetics, Dr. med. Galya Dragieva, 8008 Zürich (CH)
(72) Erfinder: DRAGIEVA, Galya, 8008 Zürich (CH)
(74) Vertreter: Schmauder & Partner AG Patent- & Markenanwälte VSP

(57) **Zusammenfassung**

Die Erfindung betrifft autologes,mechanisch aufbereitetes Nanofett zur Verwendung i) bei der regenerativen Behandlung von altersbedingt, lichtbedingt oder narbenbedingt geschädigter Haut einer Person, oder ii) bei der nicht-therapeutischen Verbesserung der Hautqualität und des Hauterscheinungsbildes einer gesunden Person. Dabei erfolgt eine subkutane Injektion einer ersten Portion des Nanofetts (NF) in einen ausgewählten Hautbereich (B), wobei eine Injektionskanüle (IK) durch mindestens einen Hauteintrittspunkt (E1, E2, ...) ins subkutane Gewebe (SC) eingeführt wird, danach das Nanofett retrograd und fächerförmig injiziert und durch Massage gleichmässig im Gewebe verteilt wird. Danach erfolgen flächige, intradermale Mikropunkturen mithilfe eines oszillierend wirkenden Mikronadelgeräts (MN), wobei gleichzeitig eine zweite Portion Nanofett schnell und gleichmässig in die Dermis (DE) des ausgewählten Hautbereichs (B) appliziert wird.

## Beschreibung

### Technisches Gebiet

Die Erfindung betrifft autologes, mechanisch aufbereitetes Nanofett zur Verwendung bei der Behandlung struktureller und/oder funktioneller Hautveränderungen einer Person, insbesondere zur Hautverjüngung und Gesichtsverjüngung, wobei das Nanofett mittels eines synergistischen multilayer Applikationskonzepts mit kombinierter subkutaner und oszillierend microneedling-assistierter intradermaler Verabreichung appliziert wird.

Ferner betrifft die Erfindung die nicht-therapeutische Verwendung von autologem, mechanisch aufbereitetem Nanofett zur Verbesserung des Hauterscheinungsbildes und zur ästhetischen Hautverjüngung einer Person.

Ausserdem betrifft die Erfindung ein nicht-therapeutisches ästhetisches Verfahren zur Hautverjüngung und Verbesserung des Hauterscheinungsbildes durch kombinierte subkutane und oszillierend microneedling-assistierte intradermale Applikation von autologem, mechanisch aufbereitetem Nanofett.

### Stand der Technik

Der Transfer von Eigenfett in Form von Nanofett zur Anwendung an der Haut ist bereits seit längerem bekannt, siehe insbesondere Tonnard P, Verpaele A, Peeters G, Hamdi M, Cornelissen M, Declercq H. Nanofat grafting: Basic research and clinical applications. Plast Reconstr Surg. 2013; 132: 1017-26. Das Nanofett wurde durch superfizielle intradermale und subdermale Injektionen mithilfe einer 27-Gauge-Nadel appliziert. Diese Technik ermöglichte es, die Nanofat-Suspension in sehr oberflächliche Hautschichten einzubringen, insbesondere zur Verbesserung des Hauterscheinungsbildes, zur Reduktion feiner Fältchen, sowie zur Aufhellungdunkler Augenringe. Ferner wurden Anwendungen von Nanofett zur Behandlung narbiger Hautveränderungen beschrieben.

Eine Übersicht der neueren Literatur über den Nanofetttransfer findet sich bei La Padula S et al., Nanofat in Plastic Reconstructive, Regenerative, and Aesthetic Surgery: A Review of Advancements in Face-Focused Applications, J. Clin. Med. 2023, 12, 4351.

In einer Anwendungsstudie mit 50 Probanden wurde eine aus Eigenfett gewonnene Nanofettsuspension mit einer 25-G-Kanüle in die Subkutanschicht an verschiedenen Gesichtsarealen injiziert, siehe Menkes S. et al., Subcutaneous Injections of Nanofat Adipose-derived Stem Cell Grafting in Facial Rejuvenation, Plast Reconstr Surg Glob Open, 2020 Jan 20; 8(1): e2550. Bei sämtlichen Probanden waren die klinischen Ergebnisse zwischen 2 und 4 Wochen nach der Injektion sichtbar, und Verbesserungen wurden kontinuierlich über die Zeit hinweg bis zu 6 Monate postoperativ beobachtet.

Verpaele et al. beschrieben 2019 eine neuartige Technik zur gleichmässigen Applikation von Nanofett in die Haut mithilfe von Mikroneedling. Dabei wird das Nanofett durch ein spezielles Needling-System flächig in die Haut eingebracht. Die Methode basiert auf bestehenden Erfahrungen mit der transdermalen Verabreichung von Substanzen mittels Mikroneedling und soll die Hautqualität durch eine möglichst gleichmässige Verteilung von Nanofett verbessern. Die Autoren setzten dabei das Gerät HydraNeedle 20 (Guangzhou Ekai Electronic Technology Co., Ltd., China) ein, das mit 20 goldbeschichteten Titan-Mikronadeln (Länge 1,5 mm, Durchmesser 0,13 mm) ausgestattet ist. Durch sanften Druck auf das Applikationssystem wird die Nanofett-Suspension auf die Hautoberfläche abgegeben und gelangt über die frisch erzeugten Mikrokanäle in die oberflächlichen Hautschichten. Siehe Verpaele, A., Tonnard, P., Jeganathan, C., & Ramaut, L. (2019). Nanofat Needling: A Novel Method for Uniform Delivery of Adipose-Derived Stromal Vascular Fraction into the Skin. Plastic and Reconstructive Surgery, 143(4), 1062-1065.

Eine als "fast nanofat microneedling" bezeichnete Weiterentwicklung beruht auf der Kombination einer stempelförmigen und einer rollenförmigen Mikronadelvorrichtung, mit der angeblich eine erhebliche Beschleunigung des Gesamtverfahrens möglich wird, siehe Righesso R et al., A Combined Approach for Fast Nanofat Microneedling, J Cutan Aesthet Surg. 2021 Apr-Jun; 14(2): 248-255.

Als weitere Behandlungsmethode wurde eine Dreierkombination aus CO2-Laser-behandlung, Microneedling mit einem oszilierenden Microneedling Gerät und topische Nanofettapplikation ("LaMiNa") vorgeschlagen, siehe Claytor R, LaMiNa: A Creative Synergistic Approach to Facial Rejuvenation, Aesthet Surg J - Open Forum 2023, 27:5:ojad028.

Trotz wachsender wissenschaftlicher Evidenz für das regenerative Potenzial von Nanofett bleibt dessen breite klinische Anwendung bislang limitiert. In der täglichen klinischen Praxis spielen Effizienz, Praktikabilität und kurze Ausfallzeiten eine zentrale Rolle. Ästhetische Verfahren wie chemische Peelings, injizierbare Substanzen oder energiebasierte Verfahren werden häufig eingesetzt. Diese adressieren die Haut- und Gesichtsalterung jedoch meist nur partiell oder erfordern wiederholte Behandlungssitzungen. Nanofettbasierte Ansätze konnten bereits Fortschritte zeigen, sind jedoch bislang in ihrer breiten klinischen Anwendung limitiert oder mit erhöhtem Behandlungsaufwand verbunden. Selbst chirurgische Verfahren, bei denen Hautqualität und Gesichtskontur kombiniert behandelt werden, wie beispielsweise Facelift in Kombination mit Fetttransfer, lassen hinsichtlich Regenerationspotenzial, Effizienz und Behandlungsaufwand weiteres Verbesserungspotenzial erkennen. Es besteht daher weiterhin Bedarf an einem ganzheitlichen minimal-invasiven Ansatz, der mehrere Aspekte der Haut- und Gesichtsalterung in einer kurzen Behandlungssitzung adressiert, dabei Patientendiskomfort und Ausfallzeit minimiert und zugleich für eine breite Anwendung in der täglichen klinischen Praxis geeignet ist. Ein solcher Ansatz stellt einen weiteren Schritt in Richtung einer nachhaltigen, regenerativen ästhetischen Medizin dar.

### Darstellung der Erfindung

Aufgabe der Erfindung ist es demnach, einen verbesserten Ansatz zur ästhetischen und regenerativen Hautverjüngung bereitzustellen.

Im vorliegenden Zusammenhang bezeichnet "ästhetisch" die Verbesserung des Erscheinungsbildes der Haut und/oder des Gesichts, insbesondere im Hinblick auf Hautqualität, Hauttextur, Hautelastizität und Kontur.

Darüber hinaus können mit der erfindungsgemässen Anwendung auch regenerative und therapeutische Effekte erzielt werden, insbesondere bei strukturellen oder funktionellen altersbedingten Hautveränderungen.

Gelöst wird diese Aufgabe durch das im Anspruch 1 definierte verwendungsgebundene Produkt sowie durch das im Anspruch 6 definierte nicht-therapeutische Verfahren.

Vorteilhafte Ausführungsformen der Erfindung sind in den abhängigen Ansprüchen definiert.

Ein erster Aspekt betrifft autologes, mechanisch aufbereitetes Nanofett zur Verwendung i) bei der regenerativen Behandlung von altersbedingt, lichtsbedingt oder narbenbedingt geschädigter Haut einer Person, oder, bei der nicht-therapeutischen Verbesserung der Hautqualität und des Hauterscheinungsbildes einer gesunden Person, wobei das Nanofett mittels einer kombinierten synergistischen multilayer Applikation verabreicht wird, welche Applikation umfasst:
a) subkutane Injektion einer ersten Portion des Nanofetts (NF) in einen ausgewählten Hautbereich (B), wobei eine Injektionskanüle (IK) durch mindestens einen Hauteintrittspunkt (E1, E2, ...) ins subkutane Gewebe (SC) eingeführt wird, danach das Nanofett retrograd und fächerförmig injiziert und durch Massage gleichmässig im Gewebe verteilt wird, gefolgt von
b) flächigen, intradermalen Mikropunkturen mithilfe eines oszillierend wirkenden Mikronadelgeräts (MN), wobei gleichzeitig eine zweite Portion Nanofett schnell und gleichmässig in die Dermis (DE) des ausgewählten Hautbereichs (B) appliziert wird.

Ein weiterer Aspekt betrifft ein nicht-therapeutisches ästhetisches Verfahren zur Verbesserung des Erscheinungsbildes der Haut einer einer Person, welches die folgenden Schritte umfasst:
a) Entnahme von Eigenfett der besagten Person und mechanische Aufarbeitung des entnommenen Eigenfetts zur Herstellung von autologem, mechanisch aufbereitetem Nanofett;
b) subkutane Injektion einer ersten Portion des autologen Nanofetts in einen ausgewählten Hautbereich; eine Injektionskanüle wird dabei durch mindestens einen Hauteintrittspunkt ins subkutane Gewebe eingeführt, danach wird das Nanofett retrograd und fächerförmig injiziert und durch sanfte Massage gleichmässig im Gewebe verteilt; gefolgt von
c) flächigen, intradermalen Mikropunkturen mithilfe eines oszillierend wirkenden Mikronadelgeräts, wobei gleichzeitig eine zweite Portion Nanofett topisch appliziert wird und durch die zahlreichen und schnell erzeugten Mikrokanäle eine gleichmässige Verteilung in der Dermis ermöglicht wird.

Im vorliegenden Zusammenhang bezeichnet "ästhetisches Verfahren" ein nicht-therapeutisches Verfahren zur Verbesserung des Erscheinungsbildes der Haut und/oder des Gesichts.

Im vorliegenden Zusammenhang wird als "retrograd und fächerförmig" eine Injektionstechnik bezeichnet, bei der das Nanofett durch langsames Zurückziehen der in die Subkutis eingeführten Injektionskanüle strangförmig abgelegt. Danach erfolgt in wiederholter Folge eine azimutale Drehung der Injektionskanüle, ein erneuter Vorschub derselben und wieder ein Zurückziehen unter Bildung eines weiteren Nanofettstrangs. Nach endgültigem Rückzug der Injektionskanüle und Anwendung einer sanften Massage ergibt sich in der Subkutis eine weitgehend homogene Verteilung des Nanofetts.

Das erfindungsgemässe ästhetische Verfahren ist grundsätzlich zur Hautverjüngung jeglicher Hautpartien geeignet. Insbesondere zählen dazu die sonnenexponierten Areale wie Gesicht, Hals, Décolleté und Handrücken.

Gemäss einer vorteilhaften Ausführungsform (Anspruch 2 und 8) ist der ausgewählte Hautbereich das Gesicht. In diesem Fall sind mindestens 2 Injektionspunkte, und zwar einer pro Gesichtsseite notwendig, damit das Ergebnis möglichst symmetrisch ist.

Das erfindungsgemässe ästhetische Verfahren zur Hautverjüngung einer Person, welches auf einem autologen Nanofetttransfer beruht, ist eine sichere und effiziente Methode. Insbesondere erlaubt diese
- eine effiziente Behandlung von Hautpartien wie dunklen und z. T. eingefallenen Augenringen,
- eine Verbesserung der Hauttextur und Gesichtsform wie sie bei der Person in den früheren Jahren bestand,
- eine Aufhellung von Pigmentierung,
- langfristige Ergebnisse,
- regenerative Effekte bis 3 Jahre nach der Behandlung.

Für die Entnahme von Eigenfett im Schritt a) des Verfahrens wird zweckmässigerweise die subgluteale Region verwendet. Dies hat einerseits den Vorteil, dass auch bei sehr schlanken Personen genügend Eigenfett vorliegt. Andererseits sind dort etwaige Unregelmässigkeiten, die sich bei der Entnahme ergeben, kaum sichtbar. Für die Entnahme wird ein geringes Lipoaspiratvolumen verwendet, insbesondere im Bereich von 20 bis 40 cm³. Dies ermöglicht eine effiziente Gewinnung von Nanofett bei gleichzeitig reduzierter Invasivität und erleichtert die breite klinische Anwendung im Alltag. Zum Harvesting wird vorzugsweise eine 2.1 mm Kanüle mit einer Länge von 13 cm verwendet. Diese wird über einen 3-Wege-Hahn mit einer 20 cm³ Spritze verbunden, so dass die Aspiration und Weiterverabreitung des Eigenfetts in einem geschlossenen System erfolgen kann. Dies reduziert Kontaminationsrisiko, erleichtert die standardisierte Handhabung und unterstützt eine effiziente klinische Anwendung. Die Spritze lässt sich mittels eines Johnnie Snap^{™} Einweg-Spritzenverschlusses in zwei Hälften à 10 cm³ aufteilen , wasden Aufbau eines Niedervakuums erlaubt. Nach der Entnahme erfolgt einen Spülschritt mit steriler physiologischer Kochsalzlösung (ca. 20 cm³) über den 3-Wege-Hahn zur Entfernung von Blutbestandteilen und öligen Rückstände.

In einer Ausführungsform kann das zur Herstellung des Nanofetts verwendete autologe Fettgewebe entweder unmittelbar nach der Entnahme oder nach zwischenzeitlicher Konservierung, insbesondere Kryokonservierung, verwendet werden. Dies ermöglicht eine zeitlich flexible Verwendung des Nanofetts und kann bei wiederholten Anwendungen eine erneute Fettentnahme und die damit verbundene zusätzliche Belastung für den Patienten vermeiden.

Die in den Schritten b) und c) erfolgende Kombination aus einer subkutanen Injektion gefolgt von einer flächigen Behandlung mit einem oszillierend wirkenden Mikronadelgerät und gleichzeitiger topischer Applikation von Nanofett stellt einen synergistischen, mehrschichtigen Ansatz zur Hautverjüngung - insbesondere im Gesichtsbereich - dar. Die kombinierte subkutane und intradermale Anwendung sowie die mikromechanische Stimulation durch das Needling können sich gegenseitig in ihrer regenerativen Wirkung verstärken und die Effekte der Einzelmethoden potenziell übertreffen.

Für die subkutane Injektion im Schritt b) des Verfahrens, welche durch mindestens einen Hauteintrittspunkt im ausgewählten Hautbereich bzw. mindestens einen Hauteinstichpunkt pro Gesichtsseite erfolgt, werden vorzugsweise Injektionskanülen im Grössenbereich 22G bis 25G verwendet. Je nach Situation können auch mehr Hauteintrittspunkte, beispielsweise 2 bis 4 oder sogar noch mehr verwendet werden.

Die flächige Behandlung im Schritt c) des Verfahrens erfolgt mit einem oszillierend wirkenden Mikronadelgerät, auch als Needling-System bezeichnet, welches eine Mehrzahl von matrixförmig angeordneten Mikronadeln umfasst, und gleichzeitiger topischer Applikation von Nanofett. Vorteilhafterweise werden damit Stichraten von ca. 1500 bis 2'500 Hz, insbesondere 1'800 bis 2'200 Hz ausgeführt.

Im Sinne der Erfindung umfasst der Begriff "oszillierend wirkendes Mikronadelgerät" alle Mikronadelgeräte, bei denen die Nadeln elektronisch gesteuert wiederholt in die Haut eindringen - unabhängig davon, ob die Bewegung kontinuierlich (klassisch oszillierend), sequentiell, impulsweise oder durch andere geeignete Mechanismen erfolgt.

Dies schliesst auch Geräte ein, die zusätzlich energiegestützt arbeiten - beispielsweise unter Verwendung von Radiofrequenz, Licht, elektromagnetischen Feldern oder anderen physikalischen Energieformen - sofern sie intradermale Mikropunkturen mittels elektronisch gesteuerter Mikronadeln erzeugen.

Ein System zur Durchführung von Radiofrequenz-Mikroneedling ist in der US 2021/0236193 A1 beschrieben.

Die "Mikroneedling-Methode" im Sinn der Erfindung könnte eine effizientere und gleichmässigere Verteilung des Nanofetts in der Dermis gewährleisten im Vergleich zu stempelförmigen oder rollenförmigen Mikronadelvorrichtungen. Eine gleichmässige Verteilung in der Dermis ist besonders wichtig, da sich dort Kollagen und Elastin bilden, die für die Regeneration und Elastizität der Haut entscheidend sind. Darüber hinaus können synergistische regenerative Effekte durch die Kombination entstehen. Zudem werden mögliche Nebenwirkungen wie Schmerzen, Rötungen oder lokale Schwellungen deutlich minimiert. Ein weiterer Vorteil liegt in der Tatsache, dass bei dieser Methode kein Risiko einer intravasalen Injektion besteht. Die Behandlung erfolgt in der Regel ohne Lokalanästhesie mittels eines oszillierend wirkenden Mikroneedling-Systems, wodurch auch die potentiell toxische Wirkung von Lokalanästhetika auf die im Nanofett enthaltenen regenerativen Zellen vollständig vermieden wird.

Bei einer vorteilhaften Ausführungsform wird ein als "Dermapen 4^{™} Microneedling Pen" im Handel erhältliches Mikronadelgerät verwendet, das in der Regel 16 Nadeln pro Kartusche umfasst. Jede Nadel hat einen Durchmesser von etwa 0.2 mm.

Diese Nadeln sind linear in einer Reihe oder leicht versetzt in einer rechteckigen Matrix angeordnet, was für eine gleichmässige Penetration im behandelten Hautbereich sorgt. Bei einer Anordnung von 4 Reihen zu 4 Nadeln mit einem Abstand zwischen benachbarten Nadeln von 1 mm ergibt sich eine Matrixfläche von ungefähr 9 mm². Die entsprechende Nadeldichte beläuft sich auf 178 Nadeln/cm².

Die Nadeln bestehen meist aus chirurgischem Edelstahl oder Titan, was sie besonders hygienisch und stabil macht.

Die Eindringtiefe der Nadeln kann präzise von 0.2 mm bis zu 3.0 mm eingestellt werden, damit je nach Hautareal und gewünschter Wirkung beste Ergebnisse erzielt werden.

Ein Vorteil dieser Nadelanordnung ist die geringere Hautbelastung bei gleichzeitig höherer Effektivität - im Vergleich zu Modellen mit weniger Nadeln.

Das Mikronadelgerät verfügt über mehrere Geschwindigkeitsstufen, die die Anzahl der Nadelstiche pro Sekunde bestimmen:
- Geschwindigkeit 1: 80 Hertz (80 Stiche pro Sekunde)
- Geschwindigkeit 2: 90 Hertz (90 Stiche pro Sekunde)
- Geschwindigkeit 3: 105 Hertz (105 Stiche pro Sekunde)
- Geschwindigkeit 4: 120 Hertz (120 Stiche pro Sekunde)

Bei einer 16-Nadel-Kartusche bedeutet dies, dass bei der höchsten Geschwindigkeit (120 Hz) insgesamt 1'920 Mikrostiche pro Sekunde erzeugt werden (16 Nadeln × 120 Stiche pro Sekunde).

Diese hohe Stichrate ermöglicht eine effiziente und effektive Mikroneedling-Behandlung mit verkürzter Behandlungszeit und minimaler Ausfallzeit.

Die erfindungsgemässe Kombination einer subkutanen Behandlung gefolgt von einer flächigen intradermalen Behandlung hat, hier aufgezählt bei der Anwendung im Gesicht, folgende Vorteile:
- ganzheitliche minimal-invasive Gesichtsverjüngung nach einer einmaligen Behandlung während 1 Stunde mit minimaler Ausfallzeit (24 Stunden),
- mehrere Aspekte der Gesichtsalterung werden in einer Sitzung angegangen, daher Harmonisierung des ganzen Gesichts in einem Schritt,
- sehr leichte vorübergehende Rötung und Schwellung im Gesicht, die in der Regel innert 24 Stunden abklingen,
- effizientere Verteilung des Nanofetts durch das elektronische Mikroneedling-Gerät und synergistische Effekte von Mikroneedling,
- lokale Betäubung lediglich an wenigen, typischerweise 3 bis 4 Eintrittspunkten im Gesicht pro Seite, wo das Nanofett subkutan injiziert wird,
- keine Blutergüsse.

Um das Ergebnis des erfindungsgemässen Verfahrens zu optimieren, kann eine zeitgleiche oder kurz aufeinanderfolgende Behandlung, hier zusammenfassend als "zeitnahe Behandlung" bezeichnet, mit autologem plättchenreichem Plasma (PRP) sinnvoll sein (Anspruch 3 und 9). Die Zugabe von PRP zielt darauf ab, die regenerative Wirkung des Nanofetts zu verstärken und sowohl die Hautqualität zu verbessern als auch einen leichten Lifting-Effekt zu erzielen. Die Kombination zeigt regenerative Vorteile, insbesondere durch die im PRP enthaltenen Wachstumsfaktoren (siehe Menkes, loc. cit.). Dazu wird der zu behandelnden Person zunächst eine Portion von beispielsweise 15 ml Blut abgenommen, aus der dann das PRP, welches sehr reich an Wachstumsfaktoren ist, gewonnen wird.

Bei einer vorteilhaften Ausführungsform (Anspruch 4 und 10) wird das Nanofett vor der Verabreichung mit 15 bis 25 Vol.-%, vorzugsweise mit 20 Vol.-% autologem PRP angereichert.

Gemäss einer alternativen Ausführungsform ohne Vormischung wird das autologe PRP unmittelbar nach den Schritten b) und/oder c) verabreicht (Anspruch 11).

Gemäss noch einer weiteren vorteilhaften Ausführungsform (Anspruch 12) erfolgt die mechanische Aufarbeitung des entnommenen Eigenfetts durch Passage durch ein geschlossenes steriles Filtersystem mit strukturierten Schneideelementen, das eine Mikronisierung des Fettgewebes bewirkt und gleichzeitig faserige Bestandteile teilweise zurückhält. Hierzu kann das kommerziell erhältliche Lipocube^{®}-System verwendet werden, bei welchem eine mechanische Mikrofragmentierung des zuvor gewonnenen Eigenfetts erfolgt. Das abgesaugte Fettgewebe wird dabei durch ein integriertes System aus mehrschichtigen Gitterstrukturen gepresst. Durch diese feinen Filtergitter wird das Gewebe progressiv zerkleinert und homogenisiert.

Gemäss noch einer weiteren vorteilhaften Ausführungsform (Anspruch 13) wird vor dem Schritt b) eine Behandlung des ausgewählten Bereichs mit einem fraktionierten Laser, insbesondere mit einem fraktionierten CO₂-Laser (Anspruch 14) durchgeführt. Dabei wird ein gebündelter Lichtstrahl in eine Vielzahl mikroskopischer Strahlen aufgeteilt, die sogenannte Mikrothermalzonen erzeugen. Ablative fraktionierte Laser wie z. B. CO₂- oder Er:YAG-Laser entfernen dabei punktuell Anteile der Epidermis und Dermis und erzeugen offene Mikrokanäle, während nicht-ablative fraktionierte Laser thermische Effekte in der Tiefe verursachen, ohne die Hautoberfläche sichtbar abzutragen. Beide Verfahren führen zu temporären Mikroverletzungen, die die Regeneration des Gewebes anregen. Diese Mikrokanäle könnten die transkutane Aufnahme von topisch appliziertem Nanofett ermöglichen bzw. verbessern und dadurch die Effektivität der intradermalen Applikation steigern. Gleichzeitig könnten die regenerativen und antiinflammatorischen Eigenschaften des Nanofetts zu einer rascheren Heilung und einer Reduktion der postprozeduralen Ausfallzeit beitragen (z. B. 3-4 Tage statt 7-10 Tage bei alleiniger CO₂-Laserbehandlung).

Diese Behandlung erfolgt im Wesentlichen unmittelbar vor dem Schritt b) oder zwischen den Schritten b) und c). Die Grundlagen einer solchen Behandlungsmodalität sind der Veröffentlichung von Claytor entnehmbar. Bei einem fraktionierten CO₂-Laser wird der austretende Lichtkegel in ein Bündel von beispielsweise 100 einzelne Strahlen aufgeteilt, die auf einer Hautfläche von 1 cm² entsprechende mikroskopische Behandlungszonen bilden. Dadurch kann im Vergleich zur herkömmlichen CO₂-Laserbehandlung eine deutlich schonendere und schneller heilende Therapie erzielt werden.

Darüber hinaus wird der fraktionierte CO₂-Laser zunehmend auch zur transdermalen Wirkstoffverabreichung eingesetzt, da die entstehenden Mikrokanäle eine gezielte Penetration von Wirkstoffen in die Dermis ermöglichen. In diesem Zusammenhang könnte der kombinierte Einsatz mit Nanofett eine besonders wirkungsvolle Anwendung darstellen: Einerseits wird durch die erzeugten Mikrokanäle eine gleichmäßigere intradermale Verteilung des Nanofetts erleichtert, andererseits könnten die antiinflammatorischen und regenerativen Eigenschaften des Nanofetts zur verbesserten Heilung und höheren Patientenzufriedenheit beitragen.

In unserer klinischen Erfahrung zeigt sich, dass die Kombination mit fraktioniertem CO₂-Laser die übliche postprozedurale Ausfallzeit nach Laserbehandlungen deutlich reduziert - von typischerweise 7-10 Tagen auf etwa 3-4 Tage.

Wie oben erwähnt, wurde 2023 von Claytor die sogenannte LaMiNa-Technik vorgestellt - ein dreistufiges regeneratives Verfahren bestehend aus fraktioniertem CO₂-Laser, Mikroneedling mittels eines oszillierenden elektronischen Geräts und topischer Applikation von Nanofett. Auch wenn in dieser Kombination synergistische regenerative Effekte und eine verkürzte Ausfallzeit beobachtet wurden, fehlt dem Ansatz der subtile volumengebende Effekt, der durch subkutane Nanofett-Injektionen erzielt werden kann.

Gerade dieser dezente Volumenaufbau ist besonders wertvoll bei der Behandlung früher Tränenrinnen-Deformitäten und dunkler Augenringe - ein Bereich, in dem konventionelle Filler häufig an ihre Grenzen stossen. Die gezielte Volumenrestauration verbessert die periorbitale Kontur und trägt wesentlich zu einem umfassenden ästhetischen Verjüngungsergebnis bei.

Gemäss einer weiteren, alternativen Ausführungsform (Anspruch 15) kann ein fraktioniertes thermomechanisches Gerät verwendet werden, das Mikrokanäle mittels kontrollierter Wärmeimpulse über eine mechanische Kontaktfläche erzeugt. Auch diese Kanäle könnten eine transkutane Aufnahme von Nanofett begünstigen und die intradermale Verteilung der Suspension verbessern.

### Kurze Beschreibung der Zeichnungen

Ausführungsbeispiele der Erfindung werden nachfolgend anhand der Zeichnungen näher beschrieben, dabei zeigen:

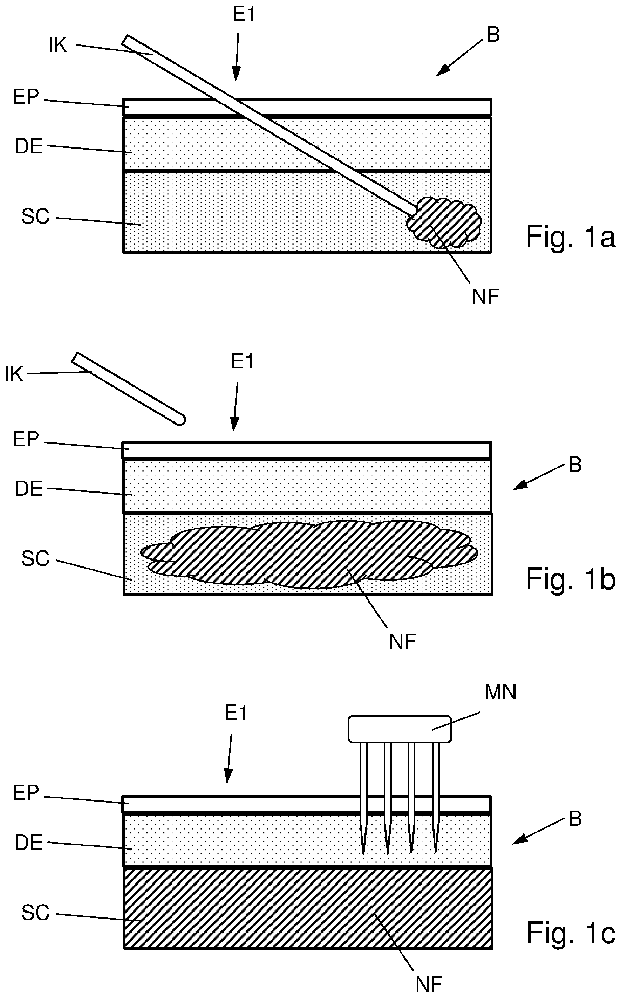

- Fig. 1: eine Darstellung des erfindungsgemässen Verfahrens anhand einer schematisch dargestellten Hautregion in einem Längsschnitt;
a) nach Einführung einer Injektionskanüle ins subkutane Gewebe und anfänglicher Einbringung von autologem Nanofett;
b) nach Rückzug der Injektionskanüle und Umverteilung des subkutan eingebrachten Nanofetts; und
c) während einer flächigen Behandlung zur intradermalen Applikation von Nanofett mittels eines oszillierend wirkenden Mikroneedling-Systems;

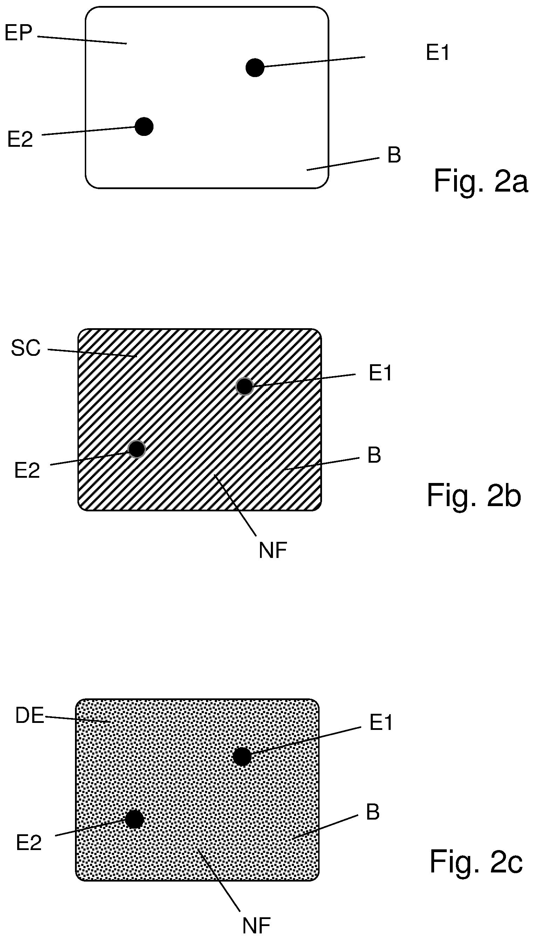

- Fig. 2: eine Darstellung des erfindungsgemässen Verfahrens anhand einer schematisch dargestellten Gesichtsregion in einer Draufsicht,
a) auf die Epidermis, unmittelbar nach Anbringung von zwei punktuellen subkutanen Injektionen;
b) auf die Subkutis, nach anschliessender Umverteilung des subkutan eingebrachten Nanofetts; und
c) auf die Dermis, nach anschliessender flächiger Behandlung durch intradermale Applikation von Nanofett mittels eines oszillierend wirkenden Mikroneedling-Systems.

### Wege zur Ausführung der Erfindung

Eine in den Fig. 1a bis 1c schematisch dargestellte Hautregion B umfasst als obere, äussere Hautschicht die Epidermis EP, unter welcher sich als angrenzende Schicht die Dermis DE und anschliessend die Subkutis SC befindet.

Die Fig. 1a zeigt die Situation nach Einführung einer Injektionskanüle IK durch einen Hauteintrittspunkt E1 bis in die Subkutis SC. Üblicherweise werden für diesen Zweck stumpfe Kanülen in einem Winkel von ungefähr 20° zur Hautoberfläche eingeführt. Nach Erreichen der Subkutis wird autologes Nanofett NF durch die Injektionskanüle befördert und in die subkutane Schicht gedrängt. Bei der retrograd fächerförmigen Technik wird das Nanofett durch langsames Zurückziehen der Injektionskanüle strangförmig abgelegt. Danach erfolgt in wiederholter Folge eine azimutale Drehung der Injektionskanüle, ein erneuter Vorschub derselben und wieder ein Zurückziehen unter Bildung eines weiteren Nanofettstrangs. Nach endgültigem Rückzug der Injektionskanüle und Anwendung einer sanften Massage ergibt sich die in Fig. 1b gezeigte Situation, wo sich in der Subkutis eine weitgehend homogene Verteilung des Nanofetts ausgebildet hat. Schliesslich wird wie in der Fig. 1c dargestellt eine flächige Behandlung durch intradermale Mikropunkturen vorgenommen. Dabei wird eine zweite Portion des Nanofetts mittels eines oszillierend wirkenden Mikronadelgeräts (MN) in die Dermis (DE) des ganzen Hautbereichs (B) eingebracht.

Das in den Fig. 2a bis 2c beispielhaft dargestellte Verfahren zur Hautverjüngung einer Person beginnt mit der Entnahme von Eigenfett der besagten Person. Dabei werden typischerweise 20 bis 40 ml Eigenfett aus einer fettreichen Körperregion, beispielsweise aus dem Abdominalbereich oder subgluteal, entnommen. In einem anschliessenden Mikronisierungsschritt erfolgt eine mechanische Aufarbeitung des entnommenen Eigenfetts unter Bildung von Nanofett.

Danach wird eine erste Portion des Nanofetts (und je nach Bedarf bei Volumendefiziten oder tiefen Falten zuerst Mikrofett) durch subkutane Injektion in einen ausgewählten Hautbereich eingebracht. Im hier gezeigten Beispiel wird eine Injektionskanüle durch zwei Hauteintrittspunkte E1, E2 im ausgewählten Hautbereich B geführt, siehe Fig. 2a.

Nach diesem Vorgang findet eine allmähliche Umverteilung des subkutan eingebrachten Nanofetts durch sanfte Massage statt. Dies ist in der Fig. 2b durch eine Schraffur veranschaulicht.

Abschliessend erfolgt eine flächige Behandlung des gesamten Hautbereichs B durch topische Applikation des Nanofetts und intradermales Mikroneedling statt. Dabei wird eine zweite Portion des Nanofetts mittels eines oszillierend wirkenden Mikroneedling-Systems auf den ganzen Hautbereich appliziert. Die angebrachten Mikroeinstiche sind in der Fig. 2c durch ein Punktemuster veranschaulicht.

### Ausführungsbeispiel 1

Eine 41-jährige Frau stellte sich mit einem insgesamt müden Erscheinungsbild vor. Sie beklagte dunkle Augenringe, störende Gesichtsfalten sowie ein Melasma im Bereich von Stirn, Wangen und Oberlippe.

Zudem litt sie unter einer Skin Picking Disorder (Dermatillomanie), die sich in wiederholt entzündeten Papeln, Exkoriationen und narbigen Hautveränderungen im Gesicht manifestierte.

Zur regenerativen Behandlung der narbigen Hautveränderungen sowie zur Verbesserung des Hautzustands wurde eine Nanofettbehandlung durchgeführt, wobei 12 ml autologes Nanofett mit 4 ml PRP (autologes plättchenreiches Plasma) zum Einsatz kamen. Zuerst wurde eine subkutane Verabreichung mittels einer 25G Injektionskanüle durch drei Hauteintrittspunkte pro Gesichtshälfte durchgeführt. Diese Injektionen wurden an folgenden Stellen angebracht: 1 ml im unteren Augenlid, 1 ml in der Wange, 1 ml in der nasolabialen Falte und 1 ml in den Marionettenfalten. Anschliessend wurde eine intradermale Nanofett-Mikroneedling-Behandlung des gesamten Gesichts durchgeführt, wobei die gesamte Prozedur innerhalb von 1 Stunde vorgenommen werden konnte. Unmittelbar nach der Behandlung traten leichte Rötung und Schwellung auf, die innerhalb 24 Stunden abklangen. Die Heilung der Entnahmestelle in der Gesässfalte verlief komplikationslos.

Nach 6 Wochen konnte die Probandin eine signifikante Verbesserung ihres Erscheinungsbildes mit einem aufgefrischten und jugendlicheren Aussehen verzeichnen. Das Melasma war deutlich weniger ausgeprägt, die dunklen Ringe unterhalb ihrer Augenringe hatten abgenommen und die entzündlichen Stellen waren verschwunden. Auch bei einer Nachkontrolle nach 18 Monaten blieb das Behandlungsergebnis stabil. Da im Beobachtungszeitraum keine weiteren ästhetischen oder medizinischen Behandlungen im Gesichtsbereich erfolgt waren, spricht dies für einen nachhaltigen regenerativen Effekt der beschriebenen Behandlung.

### Materialien und Methoden:

Tonnrad Fat Harvesting Canula
Lipocube^{™} Nanocube (ibita GmbH)
Dermapen 4^{™} (Dermapenworld)

### Ausführungsbeispiel 2

Eine 52-jährige Frau präsentierte sich mit typischen Anzeichen der Gesichtsalterung, darunter Erschlaffung der Oberlider, Falten unter den Augen, leichte Ptosis des Mittelgesichts und grobporige Haut. Autologes Nanofett wurde durch Lipoaspiration entnommen, durch zunehmend engere Verbindungsstücke getrieben und mittels eines Tulip Nanofilters filtriert. Subkutane Nanofett-Injektionen wurden in geringen Aliquoten mittels einer 25G-Kanüle in die Tränenrinne, Nasolabialfalten und Wangenregion verabreicht.

Anschliessend wurde unter Lokalanästhesie eine CO₂-Laser-Behandlung des gesamten Gesichts durchgeführt, gefolgt von einer Mikroneedling-Behandlung bis zum Erreichen von punktuellen Blutungen. Danach wurden insgesamt 15 ml Nanofett auf die laserbehandelte Haut topisch appliziert und eine weitere Mikroneedling-Behandlung zur Verbesserung der epidermalen Penetration. Schliesslich wurde nochmals Nanofett aufgetragen, nachdem die zuvor applizierte Menge absorbiert worden war.

Nach 6 Wochen zeigte die Probandin eine deutliche Straffung der periorbitalen Zone (Ober- und Unterlider), eine Milderung der Nasolabialfalten, eine verbesserte Gesichtskontur und eine sichtbare Verbesserung von Hautton, Hautstruktur und Porenverfeinerung.

### Ausführungsbeispiel 3

Eine 27-jährige Frau stellte sich mit langjährigen Beschwerden über eine ausgeprägte Tränenrinne sowie sichtbar hervortretende subkutane Fettläppchen im Unterlidbereich vor. Die Haut in dieser Region war extrem dünn und leicht durchscheinend, was dem periorbitalen Bereich ein dauerhaft müdes Erscheinungsbild verlieh. Die Probandin berichtete über eine deutliche emotionale Belastung durch dieses Erscheinungsbild. Eine Behandlung mit Hyaluronsäurefüller lehnte sie ausdrücklich ab.

Es wurde ein Mehrschichten-Nanofett-Protokoll durchgeführt. In jede infraorbitale Region wurden 0.5 mL PRP-Lipocube-Nanofett und 0.5 mL PRP-Tulip-Nanofett subkutan in retrograder Mikroaliquot-Technik mittels einer 25-G-Kanüle injiziert. Zusätzlich wurden jeweils 1.5 mL PRP-Lipocube-Nanofett pro Seite subkutan in die Jochbeinregion appliziert. Anschließend erfolgte ein Nanofett-Microneedling mit 5 mL PRP-Nanofett unter Verwendung des Dermapen 4^{™} (DermapenWorld, Australien) über das gesamte Gesicht.

Die Ausfallzeit war minimal: Die Probandin zeigte lediglich eine leichte Rötung, die innerhalb von 24 Stunden vollständig abklang. Schwellungen oder Hämatome traten nicht auf.

Nach drei Monaten zeigte sich eine deutliche Verbesserung der Tränenrinne. Die zuvor sichtbaren Fettläppchen waren weniger ausgeprägt. Die Hautqualität hatte sich insgesamt sichtbar verbessert - mit verfeinerten Poren, glatterer Textur und einer Aufhellung des gesamten Gesichts. Auch die Kontur des Mittelgesichts war harmonisiert. Die Probandin berichtete über ein gesteigertes Wohlbefinden und zeigte sich mit dem natürlichen Ergebnis sehr zufrieden. Auch bei einer Nachkontrolle nach 6 Monaten blieb das Behandlungsergebnis stabil.

## Patentansprüche

1. Autologes, mechanisch aufbereitetes Nanofett zur Verwendung
i) bei der regenerativen Behandlung von altersbedingt, lichtbedingt oder narbenbedingt geschädigter Haut einer Person,
oder
ii) bei der nicht-therapeutischen Verbesserung der Hautqualität und des Hauterscheinungsbildes einer gesunden Person,
wobei das Nanofett mittels einer kombinierten synergistischen multilayer Applikation verabreicht wird, welche Applikation umfasst:
a) subkutane Injektion einer ersten Portion des Nanofetts (NF) in einen ausgewählten Hautbereich (B), wobei eine Injektionskanüle (IK) durch mindestens einen Hauteintrittspunkt (E1, E2, ...) ins subkutane Gewebe (SC) eingeführt wird, danach das Nanofett retrograd und fächerförmig injiziert und durch Massage gleichmässig im Gewebe verteilt wird, gefolgt von
b) flächigen, intradermalen Mikropunkturen mithilfe eines oszillierend wirkenden Mikronadelgeräts (MN), wobei gleichzeitig eine zweite Portion Nanofett schnell und gleichmässig in die Dermis (DE) des ausgewählten Hautbereichs (B) appliziert wird.

2. Autologes Nanofett nach Anspruch 1, wobei der ausgewählte Hautbereich ((B) das Gesicht ist und die Injektionskanüle (IK) durch mindestens einen Hauteintrittspunkt pro Gesichtsseite in das subkutane Gewebe eingeführt wird.

3. Autologes Nanofett nach Anspruch 1 oder 2, welches mit 15 bis 25 Vol.-%, vorzugsweise mit 20 Vol.-% autologem PRP angereichert ist.

4. Autologes Nanofett nach einem der Ansprüche 1 bis 3, wobei das Nanofett aus frisch entnommenem oder zuvor konserviertem autologem Fettgewebe hergestellt ist.

5. Autologes Nanofett nach einem der Ansprüche 1 bis 4, wobei von der Person entnommenes Eigenfett mechanisch aufbereitet wird durch Passage durch ein geschlossenes steriles Filtersystem mit strukturierten Schneideelementen, was eine Mikronisierung des Fettgewebes bewirkt und gleichzeitig faserige Bestandteile teilweise zurückhält.

6. Nicht-therapeutisches ästhetisches Verfahren zur Verbesserung des Erscheinungsbildes der Haut einer Person, umfassend die folgenden Schritte:
a) Entnahme von Eigenfett der besagten Person und mechanische Aufarbeitung des entnommenen Eigenfetts zur Herstellung von autologem, mechanisch aufbereitetem Nanofett (NF),
b) subkutane Injektion einer ersten Portion des autologen Nanofetts (NF) in einen ausgewählten Hautbereich (B), wobei eine Injektionskanüle (IK) durch mindestens einen Hauteintrittspunkt (E1, E2, ...) ins subkutane Gewebe (SC) eingeführt wird, danach das Nanofett retrograd und fächerförmig injiziert und durch Massage gleichmässig im Gewebe verteilt wird, gefolgt von
c) flächigen, intradermalen Mikropunkturen mithilfe eines oszillierend wirkenden Mikronadelgeräts (MN), wobei gleichzeitig eine zweite Portion Nanofett schnell und gleichmässig in die Dermis (DE) des ausgewählten Hautbereichs (B) appliziert wird.

7. Verfahren nach Anspruch 6, wobei der ausgewählte Hautbereich ((B) das Gesicht ist und die Injektionskanüle (IK) durch mindestens einen Hauteintrittspunkt pro Gesichtsseite in das subkutane Gewebe eingeführt wird.

8. Verfahren nach Anspruch 6 oder 7, wobei zeitnah zu den Schritten b) und/oder c) eine Verabreichung von autologem PRP durchgeführt wird.

9. Verfahren nach Anspruch 8, wobei die Verabreichung von autologen PRP dadurch erfolgt, dass das verabreichte Nanofett vorgängig mit 15 bis 25 Vol.-%, vorzugsweise mit 20 Vol.-% autologem PRP angereichert wird.

10. Verfahren nach Anspruch 9, wobei die Verabreichung von autologem PRP unmittelbar nach den Schritten b) und/oder c) erfolgt.

11. Verfahren nach einem der Ansprüche 6 bis 10, wobei die mechanische Aufarbeitung des entnommenen Eigenfetts durch Passage durch ein geschlossenes steriles Filtersystem mit strukturierten Schneideelementen erfolgt, das eine Mikronisierung des Fettgewebes bewirkt und gleichzeitig faserige Bestandteile teilweise zurückhält.

12. Verfahren nach einem der Ansprüche 7 bis 11, wobei vor dem Schritt b) oder zwischen den Schritten b) und c) eine Behandlung des ausgewählten Bereichs mit einem fraktionierten Laser durchgeführt wird.

13. Verfahren nach Anspruch 12, wobei der fraktionierte Laser ein fraktionierter CO₂-Laser ist.

14. Verfahren nach einem der Ansprüche 6 bis 13, wobei vor dem Schritt b) eine Behandlung des ausgewählten Bereichs mit einem fraktionierten thermomechanischen Gerät erfolgt.
